# EUROPÄISCHE PATENTANMELDUNG

(11) **EP 1 985 990 A2**
(43) Veröffentlichungstag der Anmeldung: **29.10.2008**
(21) Anmeldenummer: 08152667.5
(22) Anmeldetag: 13.03.2008
(51) Int. Cl.: G01N 3/32, G01N 29/04, G01N 33/34

(54) **Ultraschallverfahren zur Bestimmung der Festigkeit einer Faserstoffbahn in Dickenrichtung**

(30) Priorität: 25.04.2007 DE 102007019486
(71) Anmelder: Voith Patent GmbH, 89522 Heidenheim (DE)
(72) Erfinder: Dr. Katzenmeier, Tillman, 88213, Ravensburg (DE); Rothmund, Tobias, 88512, Mengen (DE); Dr. Schmachtel, Rainer, 89518 Heidenheim (DE); Gruber-Nadlinger, Thomas, 3442, Langenrohr (AT)

(57) **Zusammenfassung**

Es wird ein Verfahren zur Bestimmung der Festigkeit einer Faserstoffbahn, insbesondere Papier- oder Kartonbahn, in Dickenrichtung beschrieben, bei dem online
- die laufende Faserstoffbahn mittels wenigstens eines Ultraschallsenders mit Ultraschallsignalen beaufschlagt wird,
- von der Faserstoffbahn beeinflusste Ultraschallsignale mittels wenigstens eines Ultraschallempfängers empfangen werden,
- anhand der vom Ultraschallsender ausgesandten ursprünglichen Ultraschallsignale und der von der Faserstoffbahn beeinflussten, vom Ultraschallempfänger empfangenen Ultraschallsignale wenigstens eine für den Elastizitätsmodul E der Faserstoffbahn repräsentative Größe erfasst wird,
- aus der wenigstens einen für den Elastizitätsmodul E der Faserstoffbahn repräsentativen Größe der Elastizitätsmodul E der Faserstoffbahn ermittelt wird und
- aus dem ermittelten Elastizitätsmodul E der Faserstoffbahn auf die Festigkeit der Faserstoffbahn in Dickenrichtung geschlossen wird.

## Beschreibung

Die Erfindung betrifft ein Verfahren sowie eine Vorrichtung zur Bestimmung der Festigkeit einer Faserstoffbahn, insbesondere Papier- oder Kartonbahn, in Dickenrichtung.

Es ist bis heute kein online einsetzbares Messverfahren zur Bestimmung der z-Festigkeit, das heißt in Dickenrichtung gegebene Festigkeit einer Papierbahn bekannt. So werden derzeit vom fertigen Tambour Papierproben genommen, die dann im Labor mittels zerstörender Messverfahren untersucht werden. Eine Überwachung während der Produktion des Tambours ist nicht möglich.

Der Erfindung liegt die Aufgabe zugrunde, ein verbessertes Verfahren sowie eine verbesserte Vorrichtung der eingangs genannten Art zu schaffen, die es gestatten, im laufenden Betrieb ohne Eingriff in die Produktion feststellen zu können, ob die produzierte Papierbahn die gewünschte Festigkeit in Dickenrichtung besitzt. Es soll also im laufenden Betrieb ohne Eingriff in die Produktion beispielsweise feststellbar sein, ob die Festigkeit in Dickenrichtung für die weitere Verarbeitung wie zum Beispiel ein Bedrucken ausreicht oder ob ein kritischer Wert unterschritten wird.

Bezüglich des Verfahrens wird diese Aufgabe nach der Erfindung dadurch gelöst, dass
- die laufende Faserstoffbahn mittels wenigstens eines Ultraschallsenders mit Ultraschallsignalen beaufschlagt wird,
- von der Faserstoffbahn beeinflusste Ultraschallsignale mittels wenigstens eines Ultraschallempfängers empfangen werden,
- anhand der vom Ultraschallsender ausgesandten ursprünglichen Ultraschallsignale und der von der Faserstoffbahn beeinflussten, vom Ultraschallempfänger empfangenen Ultraschallsignale wenigstens eine für den Elastizitätsmodul E der Faserstoffbahn repräsentative Größe erfasst wird,
- aus der wenigstens einen für den Elastizitätsmodul E der Faserstoffbahn repräsentativen Größe der Elastizitätsmodul E der Faserstoffbahn ermittelt wird und
- aus dem ermittelten Elastizitätsmodul E der Faserstoffbahn auf die Festigkeit der Faserstoffbahn in Dickenrichtung geschlossen wird.

Aufgrund dieser Ausbildung ist es nunmehr möglich, im Betrieb jederzeit Änderungen der wichtigen Papiereigenschaft "z-Festigkeit", das heißt Festigkeit in Dickenrichtung zu verfolgen und im Fall eines Absinkens des betreffenden Wertes geeignete Gegenmaßnahmen zu ergreifen, womit die Produktion von Ausschuss vermieden wird. Angesichts der nunmehr möglichen, bevorzugt berührungslosen Messung ist jederzeit nicht nur ein Messwert in der Länge, sondern auch die Bestimmung von Querprofilen möglich. Das erfindungsgemäße Verfahren stellt somit eine vorteilhafte technische Lösung für die Online-Bestimmung der "z-Festigkeit" dar.

Weiterhin können zur Online-Ermittlung von mindestens einer Zielgröße aus mindestens einem Messwert auch Informationen aus einem Datenerfassungssystem der der Herstellung der Faserstoffbahn dienenden Maschine, insbesondere Papiermaschine, verwendet werden, beispielsweise durch eine entsprechende OnView-Anbindung. Die

Bevorzugt erfolgen zumindest die Beaufschlagung der Faserstoffbahn mit Ultraschallsignalen und der Empfang der von der Faserstoffbahn beeinflussten Ultraschallsignale im laufenden Betrieb während der Herstellung der Faserstoffbahn.

Gemäß einer bevorzugten vorteilhaften Ausgestaltung des erfindungsgemäßen Verfahrens erfolgen auch die Erfassung der wenigstens einen für den Elastizitätsmodul E der Faserstoff repräsentativen Größe, die Ermittlung des Elastizitätsmoduls E der Faserstoffbahn aus dieser wenigstens einen Größe und das Schließen auf die Festigkeit der Faserstoffbahn in Dickenrichtung aus dem ermittelten Elastizitätsmodul E im laufenden Betrieb während der Herstellung der Faserstoffbahn.

In bestimmten Fällen ist es von Vorteil, wenn zumindest ein Ultraschallsender und zumindest ein Ultraschallempfänger auf einander entgegen gesetzten Seiten der Faserstoffbahn angeordnet werden. Jedoch können in weiterer Ausgestaltung zumindest ein Ultraschallsender und zumindest ein Ultraschallempfänger auch in einem einzigen Modul integriert sein, also praktisch an derselben Stelle angeordnet sein.

Es ist jedoch auch denkbar, zumindest einen Ultraschallsender und zumindest einen Ultraschallempfänger auf ein und derselben Seite der Faserstoffbahn anzuordnen. In diesem Fall ist eine in Bahnlaufrichtung versetzte Anordnung von Ultraschallsender und Ultraschallempfänger bevorzugt.

Allgemein können auch mindestens ein Ultraschallsender und zumindest ein Ultraschallempfänger in jeder beliebigen Richtung angeordnet sein. Misst man beispielsweise schräg durch die Faserstoffbahn bei einer versetzten Anordnung von zumindest einem Ultraschallsender und zumindest einem Ultraschallempfänger, so ist eine Bestimmung aller E-Module möglich. Dabei müssen bei den verschiedenen Messergebnissen die entsprechenden Anteile in x-, y- oder z-Richtung mittels vergleichenden Messungen herausgefiltert werden.

Gemäß einer zweckmäßigen vorteilhaften Ausgestaltung des erfindungsgemäßen Verfahrens werden als für den Elastizitätsmodul E der Faserstoffbahn repräsentative Größen die Schallgeschwindigkeit c in der Faserstoffbahn und die Dichte ρ der Faserstoffbahn erfasst und aus diesen Größen nach der Beziehung E ≈ ρ x c² der Elastizitätsmodul E der Faserstoffbahn ermittelt. Zur Erfassung der Schallgeschwindigkeit c und der Dichte ρ sind zweckmäßigerweise zumindest ein Ultraschallsender und zumindest ein Ultraschallempfänger auf einander entgegen gesetzten Seiten der Faserstoffbahn angeordnet.

Wenn man die Ausbreitungsgeschwindigkeit der Schallwelle über die Phasenverschiebung misst, so kann es für die Genauigkeit der Bestimmung vorteilhaft sein, die Wellenlänge zu verkleinern, das heißt die Frequenz zu erhöhen. Die Dämpfung des Ultraschallsignals durch einen Körper ist abhängig von der Streuung, Inhomogenitäten, Reflexion und Streuung an Grenzschichten, Schallfeldgeometrie und Absorbtion.

Der Absorbtionskoeffizient (in Dezibel je Längeneinheit) steigt bei allen Stoffen nahezu linear mit zunehmender Frequenz an.

Auch kann es für die Auswertung der Messergebnisse von Vorteil sein, wenn die Messungen bei verschiedenen Frequenzen durchgeführt werden. Grund hierfür sind mögliche Phasenverschiebungen und Absorbtionen.

Es könnte auch möglich sein, dass die Dichte oder zumindest eine Änderung der Dichte oder Eigenschaften der Faserstoffbahn, die auf die Dichte schließen lassen, ebenfalls durch Ultraschall festgestellt werden können, indem der Schallwiderstand (Dämpfung) durch die Faserstoffbahn bestimmt wird.

Gemäß einer weiteren vorteilhaften Ausgestaltung des erfindungsgemäßen Verfahrens werden als für den Elastizitätsmodul E der Faserstoffbahn repräsentative Größe über die Schwingung der Faserstoffbahn die Eigenfrequenz oder eine Dämpfungskonstante erfasst und daraus indirekt der Elastizitätsmodul E ermittelt. Dabei werden zur Erfassung der Eigenfrequenz bzw. der Dämpfungskonstante zweckmäßigerweise zumindest ein Ultraschallsender und zumindest ein Ultraschallempfänger auf ein und derselben Seite der Faserstoffbahn angeordnet, wobei diese in Bahnlaufrichtung bevorzugt versetzt angeordnet werden.

Mit dem erfindungsgemäßen Verfahren können vorteilhafterweise online insbesondere auch Änderungen der Festigkeit der Faserstoffbahn in Dickenrichtung erfasst werden.

Eine weitere bevorzugte Ausgestaltung des erfindungsgemäßen Verfahrens zeichnet sich dadurch aus, dass wenigstens eine der erfassten bzw. ermittelten Größen einschließlich der daraus ermittelten Festigkeit der Faserstoffbahn in Dickenrichtung über wenigstens einen Regelkreis dazu herangezogen wird, einen oder mehrere die Herstellung der Faserstoffbahn beeinflussende Parameter der der Herstellung der Faserstoffbahn dienenden Maschine, insbesondere Papiermaschine, im Hinblick auf eine Optimierung der Festigkeit in Dickenrichtung einzustellen. Eine oder mehrere der genannten Größen können somit insbesondere in einen der Regelung der Festigkeit in Dickenrichtung dienenden Regelkreis einbezogen sein.

Von Vorteil ist insbesondere auch, wenn wenigstens ein Ultraschallsender und/oder wenigstens ein Ultraschallempfänger über die Faserstoffbahn traversiert wird. Selbstverständlich können auch mehrere Ultraschallsender und/oder Ultraschallempfänger über die Breite der Faserstoffbahn stationär angebracht sein.

Die erfindungsgemäße Vorrichtung zur Bestimmung der Festigkeit einer Faserstoffbahn, insbesondere Papier- oder Kartonbahn, in Dickenrichtung umfasst entsprechend wenigstens einen Ultraschallsender, über den die laufende Faserstoffbahn online mit Ultraschallsignalen beaufschlagbar ist, wenigstens einen Ultraschallempfänger zum Empfang von von der Faserstoffbahn beeinflussten Ultraschallsignalen, Mittel zur Erfassung wenigstens einer für den Elastizitätsmodul E der Faserstoffbahn repräsentativen Größe anhand der vom Ultraschallsender ausgesandten ursprünglichen Ultraschallsignale und der von der Faserstoffbahn beeinflussten, vom Ultraschallempfänger empfangenen Ultraschallsignale, Mittel zur Ermittlung des Elastizitätsmoduls E der Faserstoffbahn aus der wenigstens einen für den Elastizitätsmodul E der Faserstoffbahn repräsentativen Größe und Mittel, um aus dem ermittelten Elastizitätsmodul E der Faserstoffbahn auf die Festigkeit der Faserstoffbahn in Dickenrichtung zu schließen. Die genannten Mittel können zumindest teilweise insbesondere einer elektronischen Auswerteeinheit oder dergleichen zugeordnet sein.

Bevorzugte Ausführungsformen der erfindungsgemäßen Vorrichtung sind in den Unteransprüchen angegeben.

Es gibt also ein über der Faserstoffbahn angebrachter Ultraschallsender Ultraschallsignale ab, die von einem Sensor oder Ultraschallempfänger, der unterhalb der Faserstoffbahn oder auch oberhalb, jedoch in Produktionsrichtung versetzt, positioniert ist, aufgefangen werden. Aus dem ursprünglichen Signal und dem empfangenen Signal kann entweder aus der Schallgeschwindigkeit c in der Faserstoffbahn mit der Dichte ρ der Elastizitätsmodul E nach der Beziehung E ≈ ρ x c² oder aus der Schwingung der Faserstoffbahn die Eigenfrequenz oder eine Dämpfungskonstante bestimmt werden. Damit kann der Elastizitätsmodul E indirekt gemessen werden. Aus der bekannten Korrelation zwischen der Elastizität der Papierbahn und der Festigkeit in z-Richtung, das heißt in Dickenrichtung, kann auf die Festigkeit in Dickenrichtung geschlossen werden. Änderungen der z-Festigkeit bzw. Festigkeit in Dickenrichtung können somit insbesondere online erfasst werden.

Zur Kalibrierung kann zu Beginn und am Ende eines Tambours eine Probe genommen und im Labor untersucht werden. Der Messaufbau kann, wie bereits erwähnt, insbesondere auch über die Faserstoffbahn traversierbar ausgeführt sein, so dass im Betrieb jederzeit insbesondere auch ein Querprofil der z-Festigkeit bzw. Festigkeit in Dickenrichtung gemessen werden kann.

Mit der erfindungsgemäßen Lösung ist es nunmehr möglich, im Betrieb jederzeit Änderungen der wesentlichen Papiereigenschaft "z-Festigkeit" zu verfolgen und im Fall eines Absinkens des betreffenden Wertes geeignete Gegenmaßnahmen zu ergreifen, wodurch die Produktion von Ausschuss vermieden wird. Aufgrund der bevorzugt berührungslosen Messung ist jederzeit nicht nur ein Messwert in der Länge, sondern auch die Bestimmung von Querprofilen möglich. Mit der erfindungsgemäßen Lösung kann die z-Festigkeit nunmehr online bestimmt werden.

Die Erfindung kann auch außerhalb der Herstellung einer Faserstoffbahn, insbesondere Papier- oder Kartonbahn, verwendet werden, beispielsweise in einer Streichmaschine (Veredelung) oder in einer Druckmaschine (Weiterverarbeitung). Hier wirkt es sich ja gerade aus, wenn die bestimmte Eigenschaft zu schlecht ist. Eine Veränderung der Qualität der Faserstoffbahn ist dann zwar nicht mehr möglich, zur Kontrolle oder zur eventuellen Steuerung, welche Bereiche der Faserstoffbahn beispielsweise nicht verwendbar oder nicht bedruckbar sind, kann die Verwendung der Erfindung aber hilfreich sein.

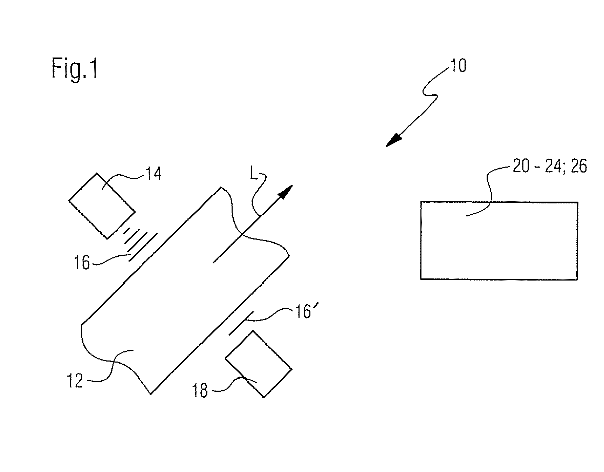

Die Erfindung wird im Folgenden anhand eines Ausführungsbeispiels unter Bezugnahme auf die Zeichnung näher erläutert.

Die einzige Figur der Zeichnung zeigt in schematischer Darstellung eine Vorrichtung 10 zur Bestimmung der Festigkeit einer Faserstoffbahn 12 in Dickenrichtung. Bei der Faserstoffbahn 12 kann es sich insbesondere um eine Papier- oder Kartonbahn handeln.

Die Vorrichtung 10 umfasst wenigstens einen Ultraschallsender 14, über den die laufende Faserstoffbahn 12 online mit Ultraschallsignalen 16 beaufschlagbar ist, wenigstens einen Ultraschallempfänger 18 zum Empfang von von der Faserstoffbahn 12 beeinflussten Ultraschallsignalen 16', Mittel 20 zur Erfassung wenigstens einer für den Elastizitätsmodul E der Faserstoffbahn 12 repräsentativen Größe anhand der vom Ultraschallsender 14 ausgesandten ursprünglichen Ultraschallsignale 16 und der von der Faserstoffbahn 12 beeinflussten, vom Ultraschallempfänger 18 empfangenen Ultraschallsignale 16', Mittel 22 zur Ermittlung des Elastizitätsmoduls E der Faserstoffbahn 12 aus der wenigstens einen für den Elastizitätsmodul E der Faserstoffbahn 12 repräsentativen Größe und Mittel 24, um aus dem ermittelten Elastizitätsmodul E der Faserstoffbahn 12 auf die Festigkeit der Faserstoffbahn 12 in Dickenrichtung zu schließen.

Die genannten Mittel 20 bis 24 können zumindest teilweise einer mit dem Ultraschallsender 14 und dem Ultraschallempfänger 18 verbundenen elektronischen Auswerteeinheit 26 oder dergleichen zugeordnet sein.

Bevorzugt erfolgen sowohl die Beaufschlagung der Faserstoffbahn 12 mit Ultraschallsignalen 16 und der Empfang der von der Faserstoffbahn 12 beeinflussten Ultraschallsignale 16' als auch die Erfassung der wenigstens einen für den Elastizitätsmodul E der Faserstoffbahn 12 repräsentativen Größe, die Ermittlung des Elastizitätsmoduls E der Faserstoffbahn 12 aus dieser wenigstens einen Größe und das Schließen auf die Festigkeit der Faserstoffbahn 12 in Dickenrichtung aus dem ermittelten Elastizitätsmodul E im laufenden Betrieb während der Herstellung der Faserstoffbahn 12.

Im vorliegenden Fall sind der Ultraschallsender 14 und der Ultraschallempfänger 18 auf einander entgegen gesetzten Seiten der Faserstoffbahn 12 angeordnet.

Grundsätzlich können auch mehrere Ultraschallsender 14 und/oder mehrere Ultraschallempfänger 18 vorgesehen sein.

Denkbar ist insbesondere auch eine solche Anordnung, bei der zumindest ein Ultraschallsender 14 und zumindest ein Ultraschallempfänger 18 auf ein und derselben Seite der Faserstoffbahn 12 angeordnet sind. In diesem Fall sind Ultraschallsender 14 und Ultraschallempfänger 18 zweckmäßiger in Bahnlaufrichtung L versetzt angeordnet.

Es können insbesondere Mittel 20, 22 vorgesehen sein, um als für den Elastizitätsmodul E der Faserstoffbahn 12 repräsentative Größen die Schallgeschwindigkeit c in der Faserstoffbahn 12 und die Dichte ρ der Faserstoffbahn 12 zu erfassen und aus diesen Größen nach der Beziehung E≈ ρ x c² den Elastizitätsmodul E der Faserstoffbahn 12 zu ermitteln. In diesem Fall können Ultraschallsender 14 und Ultraschallempfänger 18 zur Ermittlung der Schallgeschwindigkeit c bzw. der Dichte ρ insbesondere auf einander entgegen gesetzten Seiten der Faserstoffbahn 12 angeordnet sein.

Es können auch beispielsweise auch Mittel 20, 22 vorgesehen sein, um als für den Elastizitätsmodul E der Faserstoffbahn 12 repräsentative Größe über die Schwingung der Faserstoffbahn 12 die Eigenfrequenz oder eine Dämpfungskonstante zu erfassen und daraus indirekt den Elastizitätsmodul E zu ermitteln.

Über die Vorrichtung 10 können online Änderungen der Festigkeit der Faserstoffbahn 12 in Dickenrichtung erfasst werden.

Die Vorrichtung 10 kann insbesondere auch wenigstens einen Regelkreis umfassen, über den wenigstens eine der erfassten bzw. ermittelten Größen einschließlich der daraus ermittelten Festigkeit der Faserstoffbahn 12 in Dickenrichtung dazu herangezogen wird, einen oder mehrere die Herstellung der Faserstoffbahn 12 beeinflussende Parameter der der Herstellung der Faserstoffbahn 12 dienenden Maschine, insbesondere Papiermaschine im Hinblick auf eine Optimierung der Festigkeit in Dickenrichtung einzustellen. Die betreffenden Größen können also insbesondere in eine Regelung der Festigkeit in Dickenrichtung einbezogen sein.

Es sind insbesondere auch solche Ausführungen der Vorrichtung 10 denkbar, bei denen wenigstens ein Ultraschallsender 14 und/oder wenigstens ein Ultraschallempfänger 18 über die Faserstoffbahn 12 traversierbar ist. Bevorzugt ist die gesamte wenigstens einen Ultraschallsender und wenigstens einen Ultraschallempfänger umfassende Messeinrichtung über die Faserstoffbahn 12 traversierbar. Damit kann online jederzeit insbesondere auch ein Querprofil der z-Festigkeit bzw. Festigkeit in Dickenrichtung gemessen werden.

### Bezugszeichenliste

- 10: Vorrichtung
- 12: Faserstoffbahn
- 14: Ultraschallsender
- 16: Ultraschallsignale
- 16': Ultraschallsignale
- 18: Ultraschallempfänger
- 20: Mittel zur Erfassung wenigstens einer für den Elastizitätsmodul repräsentativen Größe
- 22: Mittel zur Ermittlung des Elastizitätsmoduls
- 24: Mittel zur Ermittlung der Festigkeit
- 26: elektronische Auswerteeinheit

## Patentansprüche

1. Verfahren zur Bestimmung der Festigkeit einer Faserstoffbahn (12), insbesondere Papier- oder Kartonbahn, in Dickenrichtung, bei dem online
- die laufende Faserstoffbahn (12) mittels wenigstens eines Ultraschallsenders (14) mit Ultraschallsignalen (16) beaufschlagt wird,
- von der Faserstoffbahn (12) beeinflusste Ultraschallsignale (16') mittels wenigstens eines Ultraschallempfängers (18) empfangen werden,
- anhand der vom Ultraschallsender (14) ausgesandten ursprünglichen Ultraschallsignale (16) und der von der Faserstoffbahn (12) beeinflussten, vom Ultraschallempfänger (18) empfangenen Ultraschallsignale (16') wenigstens eine für den Elastizitätsmodul E der Faserstoffbahn (12) repräsentative Größe erfasst wird,
- aus der wenigstens einen für den Elastizitätsmodul E der Faserstoffbahn (12) repräsentativen Größe der Elastizitätsmodul E der Faserstoffbahn (12) ermittelt wird und
- aus dem ermittelten Elastizitätsmodul E der Faserstoffbahn (12) auf die Festigkeit der Faserstoffbahn (12) in Dickenrichtung geschlossen wird.

2. Verfahren nach Anspruch 1,
**dadurch gekennzeichnet,**
**dass** zumindest die Beaufschlagung der Faserstoffbahn (12) mit Ultraschallsignalen (16) und der Empfang der von der Faserstoffbahn (12) beeinflussten Ultraschallsignale (16') im laufenden Betrieb während der Herstellung der Faserstoffbahn (12) erfolgen.

3. Verfahren nach Anspruch 2,
**dadurch gekennzeichnet,**
**dass** auch die Erfassung der wenigstens einen für den Elastizitätsmodul E der Faserstoffbahn (12) repräsentativen Größe, die Ermittlung des Elastizitätsmoduls E der Faserstoffbahn (12) aus dieser wenigstens einen Größe und das Schließen auf die Festigkeit der Faserstoffbahn (12) in Dickenrichtung aus dem ermittelten Elastizitätsmodul E im laufenden Betrieb während der Herstellung der Faserstoffbahn (12) erfolgen.

4. Verfahren nach einem der vorhergehenden Ansprüche,
**dadurch gekennzeichnet,**
**dass** zumindest ein Ultraschallsender (14) und zumindest ein Ultraschallempfänger (18) auf einander entgegengesetzten Seiten der Faserstoffbahn (12) angeordnet werden.

5. Verfahren nach einem der vorhergehenden Ansprüche,
**dadurch gekennzeichnet,**
**dass** zumindest ein Ultraschallsender (14) und zumindest ein Ultraschallempfänger (18) auf ein und derselben Seite der Faserstoffbahn (12) angeordnet werden.

6. Verfahren nach Anspruch 5,
**dadurch gekennzeichnet,**
**dass** Ultraschallsender (14) und Ultraschallempfänger (18) in Bahnlaufrichtung (L) versetzt angeordnet werden.

7. Verfahren nach einem der vorhergehenden Ansprüche,
**dadurch gekennzeichnet,**
**dass** als für den Elastizitätsmodul E der Faserstoffbahn (12) repräsentative Größen die Schallgeschwindigkeit c in der Faserstoffbahn (12) und die Dichte ρ der Faserstoffbahn (12) erfasst werden und aus diesen Größen nach der Beziehung E≈ ρ × c² der Elastizitätsmodul E der Faserstoffbahn (12) ermittelt wird.

8. Verfahren nach einem der vorhergehenden Ansprüche,
**dadurch gekennzeichnet,**
**dass** als für den Elastizitätsmodul E der Faserstoffbahn (12) repräsentative Größe über die Schwingung der Faserstoffbahn (12) die Eigenfrequenz oder eine Dämpfungskonstante erfasst und daraus indirekt der Elastizitätsmodul E ermittelt wird.

9. Verfahren nach einem der vorhergehenden Ansprüche,
**dadurch gekennzeichnet,**
**dass** Änderungen der Festigkeit der Faserstoffbahn (12) in Dickenrichtung erfasst werden.

10. Verfahren nach einem der vorhergehenden Ansprüche,
**dadurch gekennzeichnet,**
**dass** wenigstens eine der erfassten bzw. ermittelten Größen einschließlich der daraus ermittelten Festigkeit der Faserstoffbahn (12) in Dickenrichtung über wenigstens einen Regelkreis dazu herangezogen wird, einen oder mehrere die Herstellung der Faserstoffbahn (12) beeinflussende Parameter der der Herstellung der Faserstoffbahn (12) dienenden Maschine, insbesondere Papiermaschine, im Hinblick auf eine Optimierung der Festigkeit in Dickenrichtung einzustellen.

11. Verfahren nach einem der vorhergehenden Ansprüche,
**dadurch gekennzeichnet,**
**dass** wenigstens ein Ultraschallsender (14) und/oder wenigstens ein Ultraschallempfänger (18) über die Faserstoffbahn (12) traversiert wird.

12. Vorrichtung (10) zur Bestimmung der Festigkeit einer Faserstoffbahn (12), insbesondere Papier- oder Kartonbahn, in Dickenrichtung, insbesondere zur Durchführung des Verfahrens nach einem der vorhergehenden Ansprüche, mit wenigstens einem Ultraschallsender (14), über den die laufende Faserstoffbahn (12) online mit Ultraschallsignalen (16) beaufschlagbar ist, wenigstens einem Ultraschallempfänger (18) zum Empfang von von der Faserstoffbahn (12) beeinflussten Ultraschallsignalen (16'), Mitteln (20) zur Erfassung wenigstens einer für den Elastizitätsmodul E der Faserstoffbahn (12) repräsentativen Größe anhand der vom Ultraschallsender (14) ausgesandten ursprünglichen Ultraschallsignale (16) und der von der Faserstoffbahn beeinflussten, vom Ultraschallempfänger empfangenen Ultraschallsignale (16'), Mitteln (22) zur Ermittlung des Elastizitätsmoduls E der Faserstoffbahn (12) aus der wenigstens einen für den Elastizitätsmodul E der Faserstoffbahn (12) repräsentativen Größe und Mitteln (24), um aus dem ermittelten Elastizitätsmodul E der Faserstoffbahn (12) auf die Festigkeit der Faserstoffbahn (12) in Dickenrichtung zu schließen.

13. Vorrichtung nach Anspruch 12,
**dadurch gekennzeichnet,**
**dass** zumindest die Beaufschlagung der Faserstoffbahn (12) mit Ultraschallsignalen (16) und der Empfang der von der Faserstoffbahn (12) beeinflussten Ultraschallsignale (16') im laufenden Betrieb während der Herstellung der Faserstoffbahn (12) erfolgen.

14. Vorrichtung nach Anspruch 13,
**dadurch gekennzeichnet,**
**dass** auch die Erfassung der wenigstens einen für den Elastizitätsmodul E der Faserstoffbahn (12) repräsentativen Größe, die Ermittlung des Elastizitätsmoduls E der Faserstoffbahn (12) aus dieser wenigstens einen Größe und das Schließen auf die Festigkeit der Faserstoffbahn (12) in Dickenrichtung aus dem ermittelten Elastizitätsmodul E im laufenden Betrieb während der Herstellung der Faserstoffbahn (12) erfolgt.

15. Vorrichtung nach einem der vorhergehenden Ansprüche,
**dadurch gekennzeichnet,**
**dass** zumindest ein Ultraschallsender (14) und zumindest ein Ultraschallempfänger (18) auf einander entgegengesetzten Seiten der Faserstoffbahn (12) angeordnet sind.

16. Vorrichtung nach einem der vorhergehenden Ansprüche,
**dadurch gekennzeichnet,**
**dass** zumindest ein Ultraschallsender (14) und zumindest ein Ultraschallempfänger (18) auf ein und derselben Seite der Faserstoffbahn (12) angeordnet sind.

17. Vorrichtung nach Anspruch 16,
**dadurch gekennzeichnet,**
**dass** Ultraschallsender (14) und Ultraschallempfänger (18) in Bahnlaufrichtung (L) versetzt angeordnet sind.

18. Vorrichtung nach einem der vorhergehenden Ansprüche,
**dadurch gekennzeichnet,**
**dass** Mittel (20, 22) vorgesehen sind, um als für den Elastizitätsmodul E der Faserstoffbahn (12) repräsentative Größen die Schallgeschwindigkeit c in der Faserstoffbahn (12) und die Dichte ρ der Faserstoffbahn (12) zu erfassen und aus diesen Größen nach der Beziehung E≈ ρ x c² den Elastizitätsmodul E der Faserstoffbahn (12) zu ermitteln.

19. Vorrichtung nach einem der vorhergehenden Ansprüche,
**dadurch gekennzeichnet,**
**dass** Mittel (20, 22) vorgesehen sind, um als für den Elastizitätsmodul E der Faserstoffbahn (12) repräsentative Größe über die Schwingung der Faserstoffbahn (12) die Eigenfrequenz oder eine Dämpfungskonstante zu erfassen und daraus indirekt den Elastizitätsmodul E zu ermitteln.

20. Vorrichtung nach einem der vorhergehenden Ansprüche,
**dadurch gekennzeichnet,**
**dass** Mittel vorgesehen sind, um Änderungen der Festigkeit der Faserstoffbahn (12) in Dickenrichtung zu erfassen.

21. Vorrichtung nach einem der vorhergehenden Ansprüche,
**dadurch gekennzeichnet,**
**dass** wenigstens eine der erfassten bzw. ermittelten Größen einschließlich der daraus ermittelten Festigkeit der Faserstoffbahn (12) in Dickenrichtung über wenigstens einen Regelkreis dazu herangezogen wird, einen oder mehrere die Herstellung der Faserstoffbahn (12) beeinflussende Parameter der der Herstellung der Faserstoffbahn (12) dienenden Maschine, insbesondere Papiermaschine, im Hinblick auf eine Optimierung der Festigkeit in Dickenrichtung einzustellen.

22. Vorrichtung nach einem der vorhergehenden Ansprüche,
**dadurch gekennzeichnet,**
**dass** wenigstens ein Ultraschallsender (14) und/oder wenigstens ein Ultraschallempfänger (18) über die Faserstoffbahn (12) traversierbar ist.
